# EUROPEAN PATENT APPLICATION

(11) **EP 4 591 915 A1**
(43) Date of publication of application: **30.07.2025**
(21) Application number: 24213460.9
(22) Date of filing: 16.11.2024
(51) Int. Cl.: A61M 25/10

(54) **BALLOON CATHETER**

(30) Priority: 29.01.2024 JP 2024010725
(71) Applicant: TERUMO Kabushiki Kaisha, Tokyo 151-0072 (JP)
(72) Inventor: MOTOSE, Yuji, Shizuoka, 418-0015 (JP); KOINUMA, Naoki, Shizuoka, 418-0015 (JP); SUGIKI, Tsutomu, Shizuoka,, 418-0015 (JP)
(74) Representative: KIPA AB

(57) **Abstract**

Provided is a balloon catheter (100) capable of preventing separation of a distal end cylindrical portion of a balloon (140) when the balloon catheter passes through a curved or branched blood vessel. The balloon catheter includes a cylindrical member (160), and a balloon that is disposed on a proximal side of the cylindrical member and that includes a distal side cylindrical portion (141), an actuation portion (143) having a space (140a) inflatable in a cylindrical shape, and a distal side tapered portion (142) connected between the distal side cylindrical portion and the actuation portion. In an axial cross-sectional view, a most distal end of the distal side cylindrical portion is positioned radially more inside than an outer surface of the cylindrical member, and at least the most distal end of the distal side cylindrical portion and a part of a radially inside inner surface that is radially more inside than the most distal end are positioned in contact with the cylindrical member.

## Description

### BACKGROUND OF THE INVENTION

### 1. Field of the Invention

The present invention relates to a balloon catheter.

### 2. Description of Related Art

When various medical actions are performed in a biological lumen such as a blood vessel, a balloon catheter (for example, the following Patent Literature 1) including a balloon for expanding a lesion area in the biological lumen may be used. In general, a balloon catheter is provided with a long shaft and a cylindrical member such as a soft tip disposed on a distal side of the shaft. In the balloon, a distal end neck portion (a distal side cylindrical portion) is joined to outer circumferential surfaces of the cylindrical member and a shaft inner tube (a first tube shaped body).

### Citation List

### Patent Literature

Patent Literature 1: JP2005-160536A

### SUMMARY OF THE INVENTION

In a balloon catheter disclosed in Patent Literature 1, a distal side cylindrical portion of a balloon is joined to outer circumferential surfaces of a cylindrical member and a first tube shaped body. When the balloon catheter is inserted into a curved or branched blood vessel, the balloon catheter is bent to follow a shape of the blood vessel. When a balloon of the balloon catheter is bent so as to follow the shape of the blood vessel, a welded portion between the cylindrical member and the distal side cylindrical portion may be separated. Separation of the distal side cylindrical portion of the balloon needs to be prevented as much as possible because problems such as reduction in blood vessel passage may occur.

The present invention has been made in view of the above problem, and specifically, an object of the present invention is to provide a balloon catheter capable of preventing separation of a distal end cylindrical portion of a balloon when the balloon catheter passes through a curved or branched blood vessel.

The above object of the present invention is achieved by any one of the following means (1) to (12).
(1) A balloon catheter includes a cylindrical member, and a balloon that is disposed on a proximal side of the cylindrical member and that includes a distal side cylindrical portion, an actuation portion having a space portion inflatable in a cylindrical shape, and a distal side tapered portion connected between the distal side cylindrical portion and the actuation portion, in which in an axial cross-sectional view, a most distal end of the distal side cylindrical portion is positioned radially more inside than an outer surface of the cylindrical member, and at least the most distal end of the distal side cylindrical portion and a part of a radially inside inner surface that is radially more inside than the most distal end are positioned in contact with the cylindrical member.
(2) The balloon catheter according to the above (1), in which, in the axial cross-sectional view, the cylindrical member includes a boundary portion that covers and is in continuous contact with a distal portion including the most distal end of the distal side cylindrical portion, a radially outside outer surface that is radially more outside than the most distal end, and the radially inside inner surface.
(3) The balloon catheter according to the above (2), in which the boundary portion includes a curved portion that is continuous in a curved shape via the most distal end of the distal side cylindrical portion.
(4) The balloon catheter according to the above (1), in which, in the axial cross-sectional view, the cylindrical member includes a boundary portion having a curved shape that covers and is in continuous contact with the most distal end of the distal side cylindrical portion and an outer surface distal portion that is radially more outside than the most distal end, and in a radial direction, a distance between the boundary portion and the outer surface decreases following the curved shape of the boundary portion, and disappears at an outer surface proximal end of the cylindrical member.
(5) The balloon catheter according to any one of the above (1) to (4) further includes a first tube shaped body disposed on the proximal side of the cylindrical member, in which the space portion is formed by an outer surface of the first tube shaped body and an inner surface of the actuation portion.
(6) The balloon catheter according to the above (5), in which, in the axial cross-sectional view, a distal portion of the first tube shaped body and the distal portion of the distal side cylindrical portion form an interposed portion where a part of the cylindrical member is interposed.
(7) The balloon catheter according to the above (5) or (6), in which a most distal end of the first tube shaped body is positioned closer to a distal side than the most distal end of the distal side cylindrical portion.
(8) The balloon catheter according to the above (5) or (6), in which the most distal end of the distal side cylindrical portion is positioned closer to a distal side than a most distal end of the first tube shaped body.
(9) A balloon catheter includes a cylindrical member, and a balloon that is disposed on a proximal side of the cylindrical member and that includes a distal side cylindrical portion and an actuation portion having an inflatable space portion, in which in an axial cross-sectional view, a distal portion of the distal side cylindrical portion is formed in a tongue shape protruding toward an axial distal side, and the cylindrical member closely covers the distal portion having the tongue shape on a radially inside and a radially outside.
(10) The balloon catheter according to the above (9) further includes a first tube shaped body disposed on the proximal side of the cylindrical member, in which the space portion is formed by an outer surface of the first tube shaped body and an inner surface of the actuation portion.
(11) The balloon catheter according to the above (10), in which, in the axial cross-sectional view, a distal portion of the first tube shaped body and the distal portion of the distal side cylindrical portion form an interposed portion where a part of the cylindrical member is interposed.
(12) A balloon catheter includes a cylindrical member, and a balloon that is disposed on a proximal side of the cylindrical member and that includes a distal side cylindrical portion, an actuation portion having a space portion inflatable in a cylindrical shape, and a distal side tapered portion connected between the distal side cylindrical portion and the actuation portion, in which in an axial cross-sectional view, the cylindrical member has an outer surface and an outer surface proximal end that is a proximal end on the outer surface, the distal side cylindrical portion has an outer surface and a most distal end, the most distal end of the distal side cylindrical portion is positioned radially more inside than the outer surface of the cylindrical member, and a part from the outer surface of the cylindrical member to the outer surface of the distal side cylindrical portion through the outer surface proximal end is disposed, as an outer surface of the balloon catheter, on a straight line or on a curved line protruding toward a radially outside.

According to the balloon catheter in one embodiment of the present invention, since sufficient joining strength is obtained between the cylindrical member, a shaft, and the distal side cylindrical portion of the balloon, it is possible to prevent separation of the distal side cylindrical portion of the balloon when the balloon catheter passes through a curved or branched blood vessel.

### BRIEF DESCRIPTION OF THE DRAWINGS

FIG. 1 is a view illustrating an overall configuration of a balloon catheter according to a first embodiment.
FIG. 2 is an axial cross-sectional view illustrating a periphery of a distal portion of the balloon catheter according to the first embodiment.
FIG. 3A is a partially enlarged view illustrating an A portion illustrated in FIG. 2.
FIG. 3B is a partially enlarged view illustrating a periphery of a distal side cylindrical portion.
FIG. 3C is a partially enlarged view illustrating a portion B illustrated in FIG. 3A.
FIG. 4A is a view illustrating a method (a step A) for manufacturing the balloon catheter according to the first embodiment.
FIG. 4B is a view illustrating a method (a step B) for manufacturing the balloon catheter according to the first embodiment.
FIG. 4C is a view illustrating a method (a step C) for manufacturing the balloon catheter according to the first embodiment.
FIG. 5A is a view illustrating a welding position of a distal side cylindrical portion of a balloon in a state where a cylindrical member and a first tube shaped body are connected to each other.
FIG. 5B is a partial cross-sectional view illustrating a state after balloon welding illustrated in FIG. 5A.
FIG. 6A is a view illustrating a welding position of the distal side cylindrical portion of the balloon in a state where the cylindrical member and the first tube shaped body are connected to each other.
FIG. 6B is a partial cross-sectional view illustrating a state after balloon welding illustrated in FIG. 6A.
FIG. 7A is a view illustrating a welding position of the distal side cylindrical portion of the balloon when the cylindrical member, the first tube shaped body, and the balloon are welded at one time.
FIG. 7B is a partial cross-sectional view illustrating a state after balloon welding illustrated in FIG. 7A.
FIG. 7C is a partial cross-sectional view illustrating another state after the balloon welding illustrated in FIG. 7A.
FIG. 8A is a view illustrating a welding position of the distal side cylindrical portion of the balloon when the cylindrical member, the first tube shaped body, and the balloon are welded at one time.
FIG. 8B is a partial cross-sectional view illustrating a state after balloon welding illustrated in FIG. 8A.
FIG. 8C is a partial cross-sectional view illustrating another state after the balloon welding illustrated in FIG. 8A.
FIG. 9A is a view illustrating a welding position of the distal side cylindrical portion of the balloon when the cylindrical member, the first tube shaped body, and the balloon are welded at one time.
FIG. 9B is a partial cross-sectional view illustrating a state after balloon welding illustrated in FIG. 9A.
FIG. 10 is a partial cross-sectional view illustrating a periphery of a distal portion of a balloon catheter according to a second embodiment.
FIG. 11A is a partial cross-sectional view illustrating a periphery of a distal portion according to a modification of the balloon catheter according to the first embodiment, and FIG. 11B is a partial cross-sectional view illustrating a periphery of a distal portion according to a modification of the balloon catheter according to the second embodiment.

### DESCRIPTION OF EMBODIMENTS

Hereinafter, embodiments of the present invention will be described in detail with reference to the drawings. The embodiments illustrated here are examples for embodying the technical idea of the present invention, and do not limit the present invention. In addition, other embodiments, examples, operation techniques, and the like that can be conceived by those skilled in the art without departing from the gist of the present invention are all included in the scope and the gist of the present invention and are included in the inventions described in the claims and their equivalents.

Furthermore, for convenience of illustration and understanding, the drawings attached to the present specification may be changed and schematically expressed from real objects in terms of scale, aspect ratio, shape, and the like as appropriate. These are merely examples, and are not intended to limit the interpretation of the present invention.

In the following description, when description is given by adding an ordinal number such as "first" and "second", the ordinal number is used for convenience and does not define any order unless otherwise specified. Note that in the present specification, "X to Y" indicating a range includes X and Y, and refers to "X or more and Y or less".

A configuration of a balloon catheter 100 according to a first embodiment will be described.

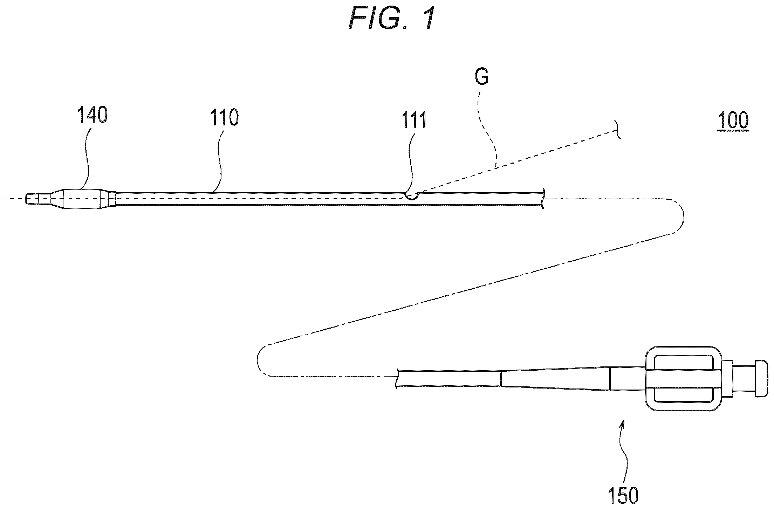

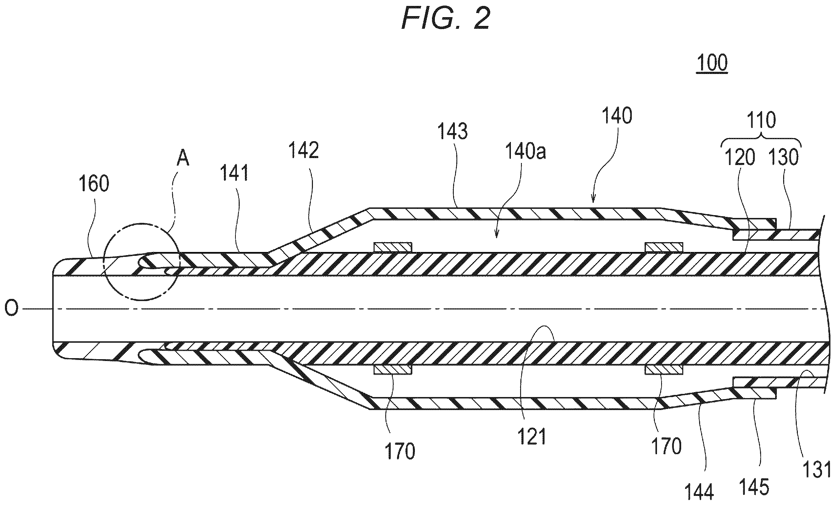

As illustrated in FIGS. 1 and 2, the balloon catheter 100 is a medical device that performs treatment by inflating a balloon 140 disposed on a distal side of a shaft 110 in a lesion area such as a stenosed site formed in a biological lumen to expand the lesion area.

The balloon catheter 100 can be configured as, for example, a balloon catheter for PTCA treatment used for expanding a lesion area of a coronary artery. Alternatively, the balloon catheter 100 can also be used for the purpose of treating and improving a lesion area formed in a biological organ such as other blood vessels, bile duct, trachea, esophagus, digestive tract, urethra, ear nasal lumen, or other organs. The balloon catheter 100 can also be used as a catheter for mounting a stent on the balloon 140 and carrying the stent to a lesion area.

In the following description, a side where the balloon 140 is disposed is referred to as a "distal side" of the balloon catheter 100, and a side where a hub 150 is disposed is referred to as a "proximal side" of the balloon catheter 100. As illustrated in FIG. 2, a direction in which a central axis O of the shaft 110 stretches is referred to as an "axial direction". A direction orthogonal to the central axis O is referred to as a "radial direction". A term "distal portion" refers to a certain range including a distal end (a most distal end) and a periphery of the distal end, and a term "proximal portion" refers to a certain range including a proximal end (a most proximal end) and a periphery of the proximal end, unless otherwise specified.

The balloon catheter 100 is configured as a so-called "rapid exchange type catheter device" in which a guide wire port 111 from which a guide wire G is led out is provided near a distal portion of the shaft 110. Note that the balloon catheter 100 may be configured as a so-called "over-the-wire type catheter device" in which a guide wire lumen 121 is formed to extend from a distal end to a proximal end of the shaft 110.

In the balloon catheter 100, as illustrated in FIG. 1, the hub 150 can be provided on a proximal portion of the shaft 110. The hub 150 can be connected to a connector (a Y connector) known in the medical field, and can be liquid-tightly and air-tightly connected to a supply device (not illustrated) such as an in-deflator for supplying a pressurized medium via the connector.

As illustrated in FIG. 2, the shaft 110 includes a first tube shaped body 120 serving as an inner tube in which the guide wire lumen 121 through which the guide wire G is inserted is formed, and a second tube shaped body 130 serving as an outer tube that forms a pressurized medium lumen 131 through which a pressurized medium can flow between the first tube shaped body 120 and the second tube shaped body 130. The shaft 110 has a double tube structure in which the first tube shaped body 120 and the second tube shaped body 130 are concentrically disposed by interpolating the first tube shaped body 120 into the second tube shaped body 130.

The balloon 140 is liquid-tightly and air-tightly joined to the distal side of the balloon catheter 100.

A cylindrical member 160 can be attached to a distal end of the first tube shaped body 120. For example, the cylindrical member 160 has a function of preventing damage to a biological organ when a distal end of the balloon catheter 100 comes into contact with the biological organ (an intravascular wall or the like). The cylindrical member 160 may be, for example, a soft tip made of a resin material softer than the first tube shaped body 120.

Imaging marker portions 170 can be provided on the first tube shaped body 120. The imaging marker portions 170 can be disposed, for example, at a position on the first tube shaped body 120 indicating a boundary with a distal side of the balloon 140, and a position on the first tube shaped body 120 indicating a boundary with a proximal side of the balloon 140.

Examples of a material of the first tube shaped body 120 and the second tube shaped body 130 include polyolefins such as polyethylene, polypropylene, ethylene-propylene copolymer, and ethylene-vinyl acetate copolymer; thermoplastic resin such as soft polyvinyl chloride; various rubbers such as silicone rubber and latex rubber; various elastomers such as polyurethane elastomer, polyamide elastomer, and polyester elastomer; and crystalline plastic such as polyamide, crystalline polyethylene, and crystalline polypropylene. It is also possible to combine these materials with an antithrombotic substance such as heparin, prostaglandins, urokinase, or arginine derivative to obtain an antithrombotic material.

The balloon 140 is disposed on the distal side of the shaft 110 (a distal side of the first tube shaped body 120).

The balloon 140 includes a distal side cylindrical portion 141, a distal side tapered portion 142 disposed adjacent to a proximal end of the distal side cylindrical portion 141, an actuation portion 143 that is disposed adjacent to a proximal end of the distal side tapered portion 142 and can be inflated and deflated by a pressurized medium, a proximal side tapered portion 144 disposed adjacent to a proximal end of the actuation portion 143, and a proximal side cylindrical portion 145 disposed adjacent to a proximal end of the proximal side tapered portion 144.

In the balloon 140, the distal side cylindrical portion 141 on the distal side is welded and joined to the first tube shaped body 120 and the cylindrical member 160, and the proximal side cylindrical portion 145 on the proximal side is welded and joined to the second tube shaped body 130.

The balloon 140 has a space portion 140a that enables the pressurized medium to flow between the balloon 140 and the first tube shaped body 120. The space portion 140a is formed by a space between an inner surface of the actuation portion 143 and an outer surface of the first tube shaped body 120. In the balloon 140, when the pressurized medium flows into the space portion 140a, the actuation portion 143 is inflated into a cylindrical shape, and when the pressurized medium flows out of the space portion 140a, the actuation portion 143 is deflated. As another aspect of the actuation portion 143, the actuation portion inflates into a spherical shape or a spindle shape. In this case, in consideration of the purpose and function of the balloon, a tapered portion may or may not be provided on a distal side and a proximal side of the actuation portion. When the balloon 140 is inflated, the balloon catheter 100 can expand and inflate a lesion area formed in a biological lumen by pressing a part of the balloon 140 against the lesion area. In a catheter in which a stent is attached to the deflated balloon 140, the stent can be indwelled due to inflation of the balloon 140, and the lesion area can be maintained in an expanded state.

A pressurized medium (for example, a fluid such as a physiological salt solution or a contrast agent) used for inflating the balloon 140 can flow into the pressurized medium lumen 131 of the shaft 110 via an internal space (a lumen) of the hub 150. The pressurized medium is supplied to the space portion 140a of the balloon 140 via the pressurized medium lumen 131.

Examples of a material of the balloon 140 include an organic polymer material. Specifically, examples of the material of the balloon 140 include a polymer material such as polyolefin (for example, polyethylene, polypropylene, polybutene, ethylene-propylene copolymer, ethylene-vinyl acetate copolymer, ionomer, or a mixture of two or more kinds thereof), polyvinyl chloride, polyamide (for example, nylon such as nylon 6, nylon 6.6, nylon 6.10, or nylon 12), polyamide elastomer, polyurethane, polyurethane elastomer, polyimide, or fluororesin, or a mixture thereof, or elastic resin such as a polymer material of two or more kinds of the above polymer materials. The balloon 140 may have a single-layer structure or a multilayer film structure including at least two layers of an inner layer and an outer layer. In the multilayer film structure, constituent materials of the inner layer and the outer layer may be the same or different.

The balloon 140 can be formed with a coating that covers an outer surface of the balloon 140. The coating can be implemented by, for example, a hydrophilic coating layer for improving a sliding property of the balloon 140 or a drug coating layer containing a predetermined drug. Specific materials for forming the hydrophilic coating layer and the drug coating layer are not particularly limited.

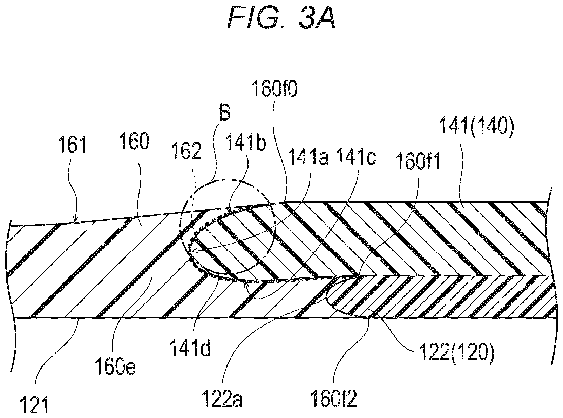

As illustrated in FIG. 3A, the balloon catheter 100 has the following feature structure in order to prevent separation (curling) of the distal side cylindrical portion 141 when the balloon catheter 100 is inserted into a curved or branched blood vessel.

In the balloon catheter 100, as illustrated in FIG. 3A, in a cross-sectional view (an axial cross-sectional view) cut along the axial direction, a most distal end 141a of the distal side cylindrical portion 141 is positioned inside the cylindrical member 160 in a manner of being radially more inside than an outer surface 161 of the cylindrical member 160. In the balloon catheter 100, in the axial cross-sectional view, an entire circumference in a circumferential direction around the axial direction of the most distal end 141a of the distal side cylindrical portion 141 is positioned radially more inside than the outer surface 161 of the cylindrical member 160, and at least the most distal end 141a of the distal side cylindrical portion 141 and a part of a radially inside inner surface 141c that is radially more inside than the most distal end 141a are positioned in contact with the cylindrical member 160. The structure illustrated in FIG. 3A can be formed by locally laser-welding the shaft 110 (the first tube shaped body 120), the balloon 140, and the cylindrical member 160 in a state where the shaft 110, the balloon 140, and the cylindrical member 160 are disposed at predetermined positions.

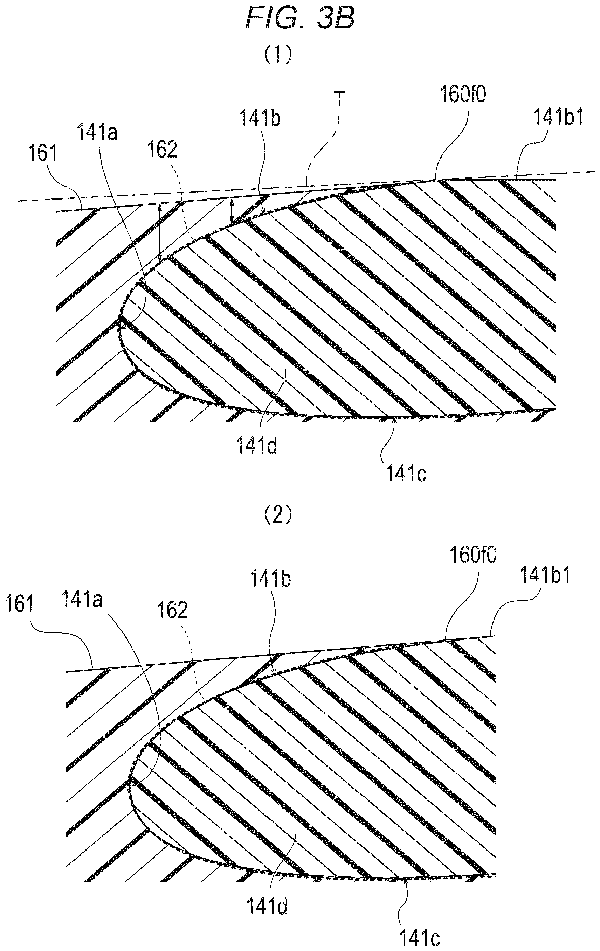

In the balloon catheter 100, as illustrated in FIG. 3A, in an axial cross-sectional view, the distal side cylindrical portion 141 includes a distal portion 141d including the most distal end 141a of the distal side cylindrical portion 141, a radially outside outer surface 141b that is radially more outside than the most distal end 141a, and the radially inside inner surface 141c that is radially more inside than the most distal end 141a. The cylindrical member 160 has a boundary portion 162 that covers and is in continuous contact with the most distal end 141a, the radially outside outer surface 141b, and the radially inside inner surface 141c. As illustrated in FIG. 3A, the radially outside outer surface 141b is a surface that is radially more outside (on an upper side in FIG. 3A) than the most distal end 141a of distal portion 141d of the distal side cylindrical portion 141 when the most distal end 141a is set as a boundary in the axial cross-sectional view. As illustrated in FIG. 3A, the radially inside inner surface 141c is a surface that is radially more inside than (on a lower side in FIG. 3A) the most distal end 141a of the distal portion 141d of the distal side cylindrical portion 141 when the most distal end 141a is set as a boundary in the axial cross-sectional view. As illustrated in FIG. 3A, the radially inside inner surface 141c has a portion of which a part is in contact with the cylindrical member 160. Further, a proximal side has a portion in contact with the distal side (the first tube shaped body 120) of the shaft 110. The boundary portion 162 with the radially outside outer surface 141b has a curved shape protruding toward a radially outside. As illustrated in (1) and (2) of FIG. 3B, in the boundary portion 162 between the radially outside outer surface 141b of the distal side cylindrical portion 141 and the cylindrical member 160, a distance between the radially outside outer surface 141b and the outer surface 161 in a radial direction decreases following a curved shape of the boundary portion 162, and the distance disappears at an outer surface proximal end 160f0 of the cylindrical member 160 (see double-headed arrows in (1) of FIG. 3B) . A decreasing rate of the distance between the boundary portion 162 and the outer surface 161 in the radial direction decreases toward an axial proximal side. The outer surface proximal end 160f0 is located on an outer surface of the balloon catheter 100.

A portion extending from the outer surface 161 of the cylindrical member 160 to an outer surface 141b1 of the distal side cylindrical portion 141 through the outer surface proximal end 160f0 is the outer surface of the balloon catheter 100, and the portion is located on a straight line or on a convex curved line that protrudes toward a radially outside. As illustrated in FIGS. 3A and (1) of FIG. 3B, the outer surface of the balloon catheter 100 from the outer surface 161 of the cylindrical member 160 to the outer surface 141b1 of the distal side cylindrical portion 141 through the outer surface proximal end 160f0 has a convex curved shape that protrudes toward a radially outside, the outer surface 161 of the cylindrical member 160 and the outer surface 141b1 of the distal side cylindrical portion 141 do not intersect with a tangent T passing through the outer surface proximal end 160f0. In other words, as long as there is no inflection point facing radially outside on an axial distal side of the outer surface 161 of the cylindrical member 160 and on an axial proximal side of the outer surface 141b1 of the distal side cylindrical portion 141, the tangent T and the outer surface 161 and the tangent T and the outer surface 141b1 are separated from each other in the radial direction as the tangent T and the outer surface 161 and the tangent T and the outer surface 141b1 are separated from the outer surface proximal end 160f0 in the axial direction. (2) in FIG. 3B illustrates an aspect in which the portion from the outer surface 161 of the cylindrical member 160 to the outer surface 141b1 of the distal side cylindrical portion 141 through the outer surface proximal end 160f0 is located on a straight line as the outer surface of the balloon catheter 100. In other words, as long as there is no inflection point facing radially outside on the axial distal side of the outer surface 161 of the cylindrical member 160 and on the axial proximal side of the outer surface 141b1 of the distal side cylindrical portion 141, the straight line does not intersect with the outer surface 161 and the outer surface 141b1.

As illustrated in FIG. 3A, the balloon catheter 100 is disposed such that the distal portion 141d of the distal side cylindrical portion 141 and a distal portion 122 of the first tube shaped body 120 are in contact with each other in the radial direction in the axial cross-sectional view. In other words, the distal portion 141d of the distal side cylindrical portion 141 and the distal portion 122 of the first tube shaped body 120 are welded to each other in a state of maintaining a boundary in the radial direction.

In the balloon catheter 100, as illustrated in FIG. 3A, a proximal portion 160e of the cylindrical member 160 is disposed in contact with the distal portion 141d of the distal side cylindrical portion 141 and the distal portion 122 of the first tube shaped body 120. Specifically, the distal portion 141d of the distal side cylindrical portion 141 and the distal portion 122 of the first tube shaped body 120 are disposed in a manner of biting into the proximal portion 160e of the cylindrical member 160. A distal side cylindrical body side proximal end 160f1 of the cylindrical member 160 is positioned between the distal portion 141d of the distal side cylindrical portion 141 and the distal portion 122 of the first tube shaped body 120. The distal portion 141d of the distal side cylindrical portion 141 and the distal portion 122 of the first tube shaped body 120 are each formed in a tongue shape having a protruding distal side toward the proximal portion 160e of the cylindrical member 160. A guide wire lumen side proximal end 160f2 of the proximal portion 160e of the cylindrical member 160 is positioned along a surface of the guide wire lumen 121, and is positioned closer to a proximal side in the axial direction than a most distal end 122a of the distal portion 122 of the first tube shaped body 120. The most distal end 141a of the distal side cylindrical portion 141 is positioned closer to a distal side in the axial direction than the most distal end 122a of the first tube shaped body 120.

The distal portion 141d of the distal side cylindrical portion 141 is formed in the tongue shape protruding toward an axial distal side in the axial cross-sectional view, and the cylindrical member 160 closely covers the distal portion 141d having the tongue shape on a radially inside and a radially outside.

As illustrated in FIG. 3A, a proximal portion of the cylindrical member 160 has the boundary portion 162 with the distal side cylindrical portion 141. In FIG. 3A, the boundary portion 162 is indicated by a thick dotted line to facilitate understanding of the drawing, but actually the boundary portion 162 coincides with an outline of the cylindrical member 160. The boundary portion 162 is a contact portion between the cylindrical member 160 and the distal side cylindrical portion 141 after welding, and has a shape surrounding at least the distal portion 141d and is in continuous contact with the distal portion 141d along an outer shape of the distal portion 141d. Accordingly, the boundary portion 162 includes a linear portion and/or a curved portion in the axial cross-sectional view. From the viewpoint of facilitating bending of the cylindrical member 160, followability to the guide wire G, and the like, the boundary portion 162 preferably includes a curved portion that is in a continuous curved shape via the most distal end 141a as illustrated in FIG. 3A.

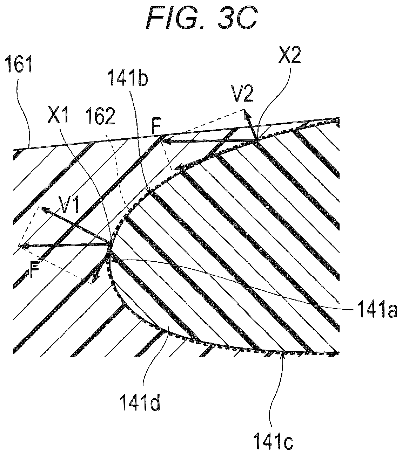

A function of the embodiment illustrated in FIG. 3A will be described. When the balloon catheter 100 is inserted along a curved blood vessel or guide wire, tensile stress is generated on an outside and compressive stress is generated on an inside of a curve. In the balloon catheter illustrated in FIG. 3A, since an angle at an intersection point X where a parallel line of the central axis O intersects with the boundary portion 162 decreases toward the radially outside, stress in a vertical direction on the intersection point X with the boundary portion 162 decreases. Actually, the tensile stress increases as moving toward the radially outside, and when it is assumed that a force F acting along the central axis O is constant from the central axis O to the outside, as illustrated in FIG. 3C, a vertical component V2 relative to a tangent of an intersection point X2 with the boundary portion 162 near the outer surface 161 is smaller than a vertical component V1 relative to a tangent of an intersection point X1 with the boundary portion 162 near the most distal end 141a of the distal side cylindrical portion 141. These vertical components are major factors of a force that separates the distal side cylindrical portion 141 from the cylindrical member 160. Further, when the boundary portion 162 is formed in a curved shape protruding toward a radially outside as illustrated in FIG. 3A, different from a linear boundary portion, a vertical component V illustrated in FIG. 3C is extremely small near the outer surface 161. Therefore, even when the cylindrical member 160 is bent, the distal side cylindrical portion 141 is unlikely to be rolled up from the cylindrical member 160.

Since the balloon catheter 100 has a "configuration in which the most distal end 141a of the distal side cylindrical portion 141 is positioned radially more inside than the outer surface 161 of the cylindrical member 160", a joining area of the distal side cylindrical portion 141 to the cylindrical member 160 can be increased. In addition, since the distal portion 141d of the distal side cylindrical portion 141 is formed in a manner of slipping into a thick portion of the cylindrical member 160 after welding, even when the distal portion 141d is bent to follow a shape of a blood vessel or a guide wire, connection with the cylindrical member 160 is firmly maintained. Accordingly, in the balloon catheter 100, since a joining strength between the cylindrical member 160 and the balloon 140 can be improved, separation of the distal portion 141d of the balloon 140 can be prevented.

Since the balloon catheter 100 has a "configuration in which the cylindrical member 160 includes the boundary portion 162 that covers and is in continuous contact with the distal portion 141d including the most distal end 141a of the distal side cylindrical portion 141, the radially outside outer surface 141b that is radially more outside than the most distal end 141a, and the radially inside inner surface 141c that is radially more inside than the most distal end 141a", a proportion of a welded portion of the distal side cylindrical portion 141 with the cylindrical member 160 increases, and a proportion of a welded portion with other members decreases. Therefore, the balloon catheter 100 easily exhibits a bending feature of the cylindrical member 160. For example, when the cylindrical member 160 is made of a material having high flexibility, flexibility of the cylindrical member 160 is improved, and the cylindrical member 160 is less likely to be caught, which makes it easier to follow a shape of a curved or branched blood vessel.

Further, in the balloon catheter 100, when the boundary portion 162 which is a welded portion (a contact portion) of the distal side cylindrical portion 141 with the cylindrical member 160 has a "configuration including a curved portion that is continuous in a curved shape via the most distal end 141a of the distal side cylindrical portion 141", a separation force caused by deformation at the time of following a blood vessel is smallest on an outer surface. Accordingly, it is possible to further enhance the effect of preventing separation of the distal portion 141d of the balloon 140. In addition, since partial stress concentration on the boundary portion 162 caused by deformation at the time of following a blood vessel can be prevented, the effect of preventing separation of the distal portion 141d of the balloon 140 can be further enhanced.

Next, a preferred embodiment of a method for manufacturing the balloon catheter 100 will be described.

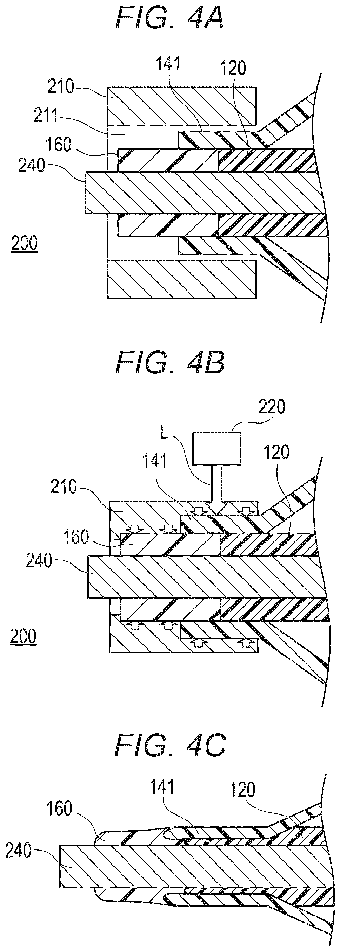

FIGS. 4A to 4C illustrate a step of joining the shaft 110, the balloon 140, and the cylindrical member 160 in the method for manufacturing the balloon catheter 100. Note that the method for manufacturing the balloon catheter 100 may include steps other than steps to be described below.

As illustrated in any one of FIGS. 4A to 4C, a manufacturing device 200 used to perform joining processing of the balloon catheter 100 includes an elastic body 210 that sandwiches an object to be joined at the time of joining so as to prevent a positional deviation of the object to be joined, and an irradiation unit 220 that irradiates the object to be joined with laser light L. Note that the manufacturing device 200 can be configured by appropriately mounting various devices necessary in a manufacturing processing, such as an operation unit and a display unit (not illustrated), in addition to the above-described configuration.

The elastic body 210 has laser light transmittance, and specifically, examples of a material of the elastic body 210 include silicone rubber and fluorine rubber. The elastic body 210 can be elastically deformed in the radial direction by pressure applied by a pressurized unit to maintain a contact state of the shaft 110, the balloon 140, and the cylindrical member 160, and joining can be efficiently performed by using the elastic body 210. Further, since the elastic body 210 has laser light transmittance, the elastic body 210 can be reused without being thermally contracted by the laser light L.

The irradiation unit 220 radiates the laser light L having a wavelength at which the laser light L causes a welded portion to generate heat due to radiation heating. A spot diameter of the laser light L may be φ0.1 mm to φ10 mm, and a wavelength of the laser light L may be 800 nm to 10000 nm. Examples of the laser light L include a fiber laser (wavelength 1070 nm), a YAG laser (wavelength 1064 nm), and a laser diode (808 nm, 840 nm, 940 nm) . When members of combined laser light absorbing materials are welded to one another, the wavelength of the laser light L can be selected from 800 nm to 5000 nm, and preferably 900 nm to 2300 nm. When transparent members are welded to one another or a member of combined laser light absorbing materials and a transparent member are welded to each other, the wavelength of the laser light L can be selected from 1300 nm to 2500 nm, and preferably 1500 nm to 2300 nm. The laser light L is basically radiated in a direction orthogonal to an axial direction of an object to be processed. The laser light L may be radiated at an angle appropriately changed depending on the object to be processed.

The balloon catheter 100 is manufactured by joining the shaft 110, the balloon 140, and the cylindrical member 160 by the manufacturing device 200. Specifically, it is preferable to include the following steps: the first tube shaped body 120 of the shaft 110, the distal side cylindrical portion 141 of the balloon 140, and the cylindrical member 160 are inserted into a hollow portion 211 of the elastic body 210 (step (A)), the elastic body 210 is pressurized on a radially inside and laser is radiated to perform heating (step (B)), pressurization is released, and the welded (joined) balloon catheter 100 is taken out (step (C)).

### Step (A)

In step (A), as illustrated in FIG. 4A, the distal side cylindrical portion 141 of the balloon 140 is disposed so as to be welded, at a predetermined welding position, to the cylindrical member 160 and the first tube shaped body 120 that are joined in advance. At the time of disposing, for example, the distal side cylindrical portion 141 of the balloon 140 can be disposed in a state of being inserted through a core 240. Accordingly, the first tube shaped body 120 of the shaft 110 and the cylindrical member 160 are movable and rotatable integrally with the core 240. The core 240 is made of, for example, metal. Note that, as will be described later, the first tube shaped body 120 of the shaft 110 and the cylindrical member 160 may not be welded in advance, and a contact portion may be formed in a state where a distal end position of the distal side cylindrical portion 141 of the balloon 140 is disposed with reference to the vicinity of a portion to be welded of the first tube shaped body 120 of the shaft 110 and the cylindrical member 160.

As illustrated in FIG. 4A, in a state where rotation axes of the core 240 and the elastic body 210 are aligned, either one of the core 240 and the elastic body 210 is moved in an axial direction to dispose the first tube shaped body 120, the cylindrical member 160, and the distal side cylindrical portion 141 in the hollow portion 211 of the elastic body 210. At this time, the cylindrical member 160 and the distal side cylindrical portion 141 do not come into contact with an inner surface of the hollow portion 211 of the elastic body 210 in the radial direction, and a gap is generated.

### Step (B)

Next, in step (B), as illustrated in FIG. 4B, the cylindrical member 160 and the distal side cylindrical portion 141 are pressurized in the radial direction. An external force (a force toward the central axis O) is applied to the elastic body 210 by a pressurized unit (not illustrated). The external force applied to the elastic body 210 presses the cylindrical member 160 and the distal side cylindrical portion 141 in directions of arrows illustrated in FIG. 4B.

As described above, positioning can be performed with high accuracy by applying pressure to the elastic body 210 at the time of heating without applying pressure at the time of setting in step A, and the effect of improving quality and reproducibility of welding is exhibited. In addition, since there is a clearance between the object to be processed and the elastic body 210, welding can be performed without affecting the object to be processed when the object to be processed is inserted into the elastic body 210. Further, it is possible to form a welded portion of the object to be processed into a taper shape, a two-stage taper shape, a gentle R shape, a local constricted portion, or the like by giving a desired shape to the elastic body 210, the pressurized unit, or the like.

Next, in step B, a welded point is irradiated with the laser light L while maintaining a state where the external force is applied. Since the elastic body 210 has laser light transmittance, the welded point is directly heated. When the laser light L is locally radiated to a contact portion, only the welded point and a periphery of the welded point can be locally heated. At the time of heating, the first tube shaped body 120 of the shaft 110, the balloon 140, and the cylindrical member 160 are rotated around the central axis O by rotating the core 240 and the elastic body 210 with the central axis O as a rotation axis, so that the heating is uniformly performed. Note that the object to be joined may not be rotated, and the irradiation unit 220 that radiates the laser light L may be rotated with the central axis O as a rotation axis.

The laser light L is locally radiated to an end surface portion of the contact portion and a peripheral portion of the end surface portion. By radiating the laser light L in such a manner, a welded portion is formed by welding a contact portion of a proximal side of the cylindrical member 160, the distal side of the shaft 110, and the distal side cylindrical portion 141 of the balloon 140 by heat generated by the shaft 110, the balloon 140, and the cylindrical member 160, and further, by heat transfer from a heat-generating member to another member (for example, when the shaft 110 generates heat, the another member is the balloon 140 and/or the cylindrical member 160). It is preferable that at least one of the shaft 110, the balloon 140, and the cylindrical member 160, more preferable at least the shaft 110, includes a laser light absorbing material, so that the object to be joined is locally heated, and since the elastic body 210 in the periphery has laser light transmittance, the elastic body 210 is not heated. Heat is dissipated to the elastic body and a peripheral portion (for example, the core 240) from the moment when the welded portion is heated, and thus the object to be joined are cooled rapidly after the radiation of the laser light is stopped.

### Step (C)

In step (C), as illustrated in FIG. 4C, the welded balloon catheter 100 can be taken out from the elastic body 210 by releasing the pressure applied to the elastic body 210 by the pressurized unit. Thereafter, the balloon catheter 100 is finished through a step of forming a lubricating coating layer on a predetermined outer circumferential surface.

Next, an arrangement aspect of each member during manufacturing the balloon catheter 100 and a structure after welding will be described. Each of the following structures can be formed by locally laser-welding the welded portion as illustrated in FIGS. 4A to 4C.

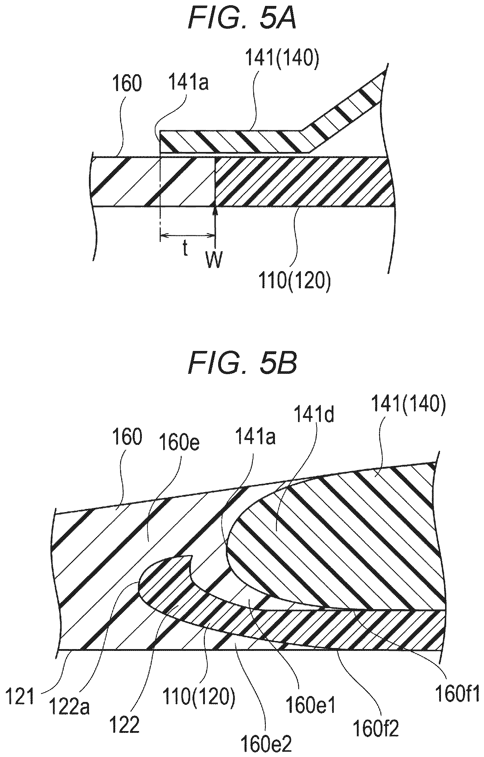

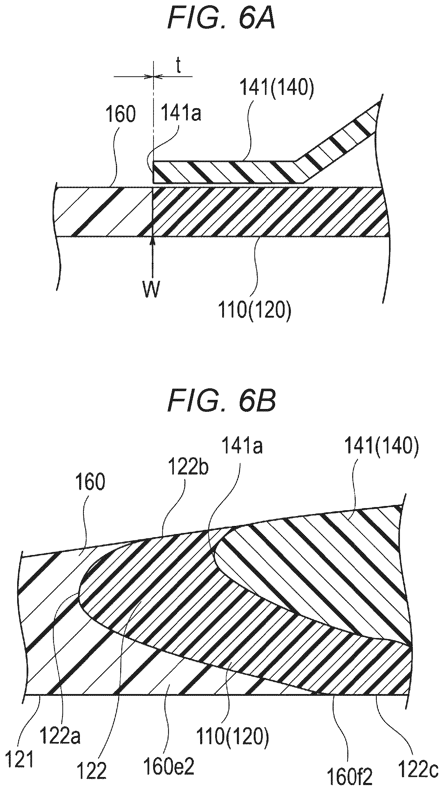

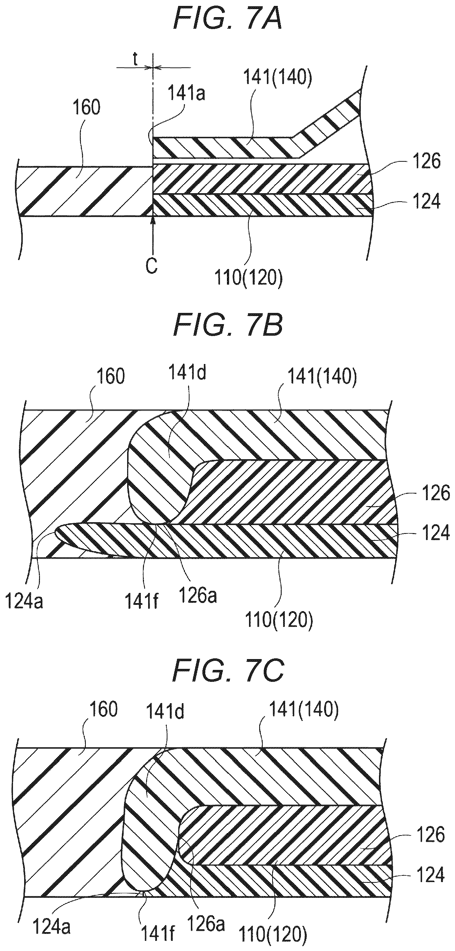

FIGS. 5A, 5B, 6A, and 6B illustrate a two-stage welding aspect in which the cylindrical member 160 and the shaft 110 (the first tube shaped body 120) are welded in advance and then the balloon 140 is welded. FIGS. 7A to 7C, 8A to 8C, 9A, and 9B illustrate an aspect in which the cylindrical member 160, the shaft 110 (the first tube shaped body 120), and the balloon 140 are welded at the same time.

The balloon catheter 100 can control a welded structure in the vicinity of the contact portion of the shaft 110, the balloon 140, and the cylindrical member 160 to a desired shape by controlling an arrangement position or the like of the distal side cylindrical portion 141 of the balloon 140 as described below.

FIG. 5A is a view illustrating an aspect in which the most distal end 141a of the distal side cylindrical portion 141 of the balloon 140 is positioned closer to the distal side than a welding position W between a proximal end of the cylindrical member 160 and the distal end of the shaft 110, that is, an aspect in which a covering length of the distal side cylindrical portion 141 of the balloon 140 on the cylindrical member 160 is set on the distal side from the welding position W (a covering length t > 0). FIG. 5B is a cross-sectional view illustrating the vicinity of a distal portion of the balloon catheter 100 manufactured by laser-welding the balloon 140 in the arrangement aspect illustrated in FIG. 5A.

In the balloon catheter 100, as illustrated in FIG. 5A, when the covering length t of the distal side cylindrical portion 141 of the balloon 140 on the cylindrical member 160 at the time of welding is set to be larger than zero, as illustrated in FIG. 5B, a flow to a proximal side of the distal side cylindrical portion 141 in the cylindrical member 160 is deep. Specifically, a distal side cylindrical body side proximal portion 160e1 is interposed between the distal portion 141d of the distal side cylindrical portion 141 and the distal portion 122 of the first tube shaped body 120. A distance between the distal portion 141d and the distal portion 122 in the distal side cylindrical body side proximal portion 160e1, that is, a thickness in the radial direction of the distal side cylindrical body side proximal portion 160e1 decreases toward an axial proximal end, and the distance disappears at the distal side cylindrical body side proximal end 160f1. A guide wire lumen side proximal portion 160e2 is positioned between the distal portion 122 of the first tube shaped body 120 and a surface of the guide wire lumen 121. A thickness in the radial direction of the guide wire lumen side proximal portion 160e2 decreases toward the axial proximal end, and the thickness disappears at the guide wire lumen side proximal end 160f2. The most distal end 141a of the distal side cylindrical portion 141 is positioned closer to the proximal side in the axial direction than the most distal end 122a of the first tube shaped body 120. When the thickness of the balloon 140 is small, since heat is not applied to a contact portion in laser-weld unlike in general heat-weld, the flow in the cylindrical member 160 is less likely to occur.

FIG. 6A is a view illustrating an aspect in which the welding position W between the proximal end of the cylindrical member 160 and the distal end of the shaft 110 is located at the same position with the most distal end 141a of the distal side cylindrical portion 141 of the balloon 140, or is located on the proximal side, that is, an aspect in which the covering length t of the distal side cylindrical portion 141 of the balloon 140 on the cylindrical member 160 is zero or the covering length is negative (the covering length t ≤ 0). FIG. 6B is a cross-sectional view illustrating the vicinity of the distal portion of the balloon catheter 100 manufactured by laser-welding the balloon 140 in an arrangement aspect illustrated in FIG. 6A.

In the balloon catheter 100, as illustrated in FIG. 6A, when the covering length t of the distal side cylindrical portion 141 of the balloon 140 on the cylindrical member 160 at the time of welding is zero or negative, it is possible to manufacture a balloon catheter having a structure in which the distal portion of the shaft 110 is interposed between the cylindrical member 160 and the distal side cylindrical portion 141 of the balloon 140 as illustrated in FIG. 6B. Specifically, the distal portion 122 of the first tube shaped body 120 of the shaft 110 forms an inner surface 122c on a radially inside on the guide wire lumen 121 side, and closely covers the most distal end 141a of the distal side cylindrical portion 141 on the axial distal side. The distal portion 122 of the first tube shaped body 120 of the shaft 110 forms an outer surface 122b on a radially outside in the radial direction. The outer surface 122b constitutes the outer surface of the balloon catheter 100. The guide wire lumen side proximal portion 160e2 is positioned between the distal portion 122 of the first tube shaped body 120 and the surface of the guide wire lumen 121. The thickness in the radial direction of the guide wire lumen side proximal portion 160e2 decreases toward the axial proximal end, and the thickness disappears at the guide wire lumen side proximal end 160f2. The most distal end 141a of the distal side cylindrical portion 141 is positioned closer to the proximal side in the axial direction than the most distal end 122a of the first tube shaped body 120.

FIG. 7A is a view illustrating a state in which the most distal end 141a of the distal side cylindrical portion 141 of the balloon 140 is positioned in the vicinity of an attach position C between the proximal end of the cylindrical member 160 and the distal end of the shaft 110, that is, a state in which the covering length t of the distal side cylindrical portion 141 of the balloon 140 on the cylindrical member 160 is almost zero (the covering length t ≈ 0) when the cylindrical member 160, the shaft 110, and the balloon 140 are welded at the same time. FIGS. 7B and 7C are cross-sectional views illustrating the vicinity of the distal portion of the balloon catheter 100 manufactured by laser-welding the cylindrical member 160, the shaft 110, and the balloon 140 in an arrangement aspect illustrated in FIG. 7A under different manufacturing conditions or using different member materials. In aspects illustrated in FIGS. 7A to 8C, the shaft 110 is a tube shaped body that is manufactured by biaxial extrusion or the like and in which an inner layer 124 and an outer layer 126 are welded and laminated in advance. In the shaft 110, a reinforcement body such as a braid or a coil may be embedded between the inner layer 124 and the outer layer 126. Such a reinforcement body may be disposed up to a most distal end of the shaft 110, or may be terminated before a point where the distal side cylindrical portion 141 of the balloon 140 is welded to the shaft 110.

As illustrated in FIG. 7A, when the balloon catheter 100 is laser-welded in a state where the covering length of the distal side cylindrical portion 141 of the balloon 140 on the cylindrical member 160 is almost zero, it is possible to manufacture a balloon catheter having a structure in which a distal side of the distal side cylindrical portion 141 enters between the shaft 110 and the cylindrical member 160 as illustrated in FIGS. 7B and 7C. Specifically, in FIG. 7B, the distal portion 141d of the distal side cylindrical portion 141 of the balloon 140 has a shape extending toward the axial distal side and then extending toward a radially inside. The distal portion 141d of the distal side cylindrical portion 141 is curved in a manner of covering a distal side of the outer layer 126. The distal portion 141d of the distal side cylindrical portion 141 is disposed in a manner of abutting against a radially outside surface of the inner layer 124 of the shaft 110. The distal portion 141d is located on the radially outside surface of the inner layer 124 as a distal side terminal end 141f. A most distal end 124a of the inner layer 124 of the shaft 110 has a structure of entering the cylindrical member 160 on the axial distal side more than the most distal end 141a of the distal side cylindrical portion 141. A most distal end 126a of the outer layer 126 of the shaft 110 terminates in a state of being interposed between the distal portion 141d of the distal side cylindrical portion 141 and an outer surface of the inner layer 124 of the shaft 110. On the other hand, in FIG. 7C, the distal portion 141d of the distal side cylindrical portion 141 of the balloon 140 has a shape extending toward the axial distal side and then extending toward a radially inside, and has a positional relationship in which the distal portion 141d covers the entire outer layer 126 of the shaft 110 and a part of the inner layer 124 of the shaft 110. That is, a distal side of the most distal end 126a of the outer layer 126 is occupied by the distal portion 141d of the distal side cylindrical portion 141. The distal side terminal end 141f of the distal side cylindrical portion 141 is in contact with the inner layer 124. The most distal end 124a of the inner layer 124 is in contact with the cylindrical member 160.

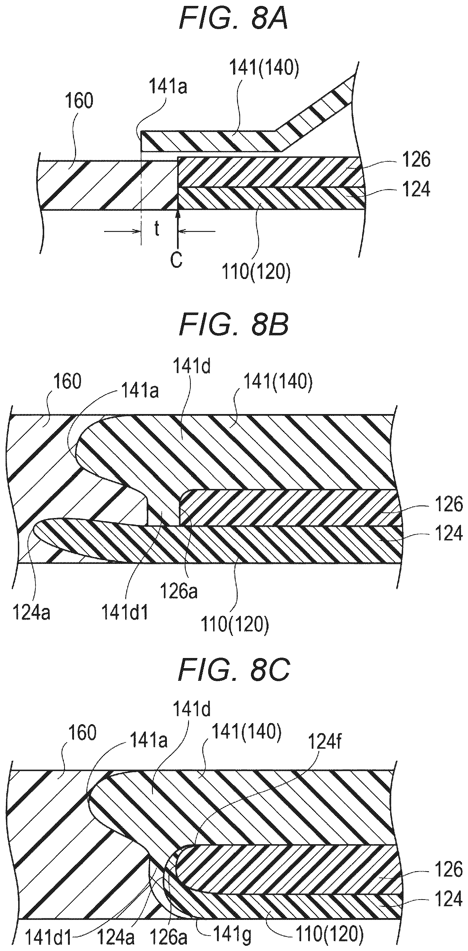

FIG. 8A is a view illustrating a state in which the most distal end 141a of the distal side cylindrical portion 141 of the balloon 140 is positioned closer to the distal side than the attach position C between the proximal end of the cylindrical member 160 and the distal end of the shaft 110, that is, a state in which the covering length t of the distal side cylindrical portion 141 of the balloon 140 on the cylindrical member 160 is long (the covering length t > 0) when the cylindrical member 160, the shaft 110, and the balloon 140 are welded at the same time. FIGS. 8B and 8C are cross-sectional views illustrating the vicinity of the distal portion of the balloon catheter 100 manufactured by laser-welding the cylindrical member 160, the shaft 110, and the balloon 140 in an arrangement aspect illustrated in FIG. 8A under different manufacturing conditions or using different member materials.

As illustrated in FIG. 8A, when the balloon catheter 100 is laser-welded in a state where the covering length t of the distal side cylindrical portion 141 of the balloon 140 on the cylindrical member 160 is long, it is possible to manufacture a balloon catheter having a structure in which a part of the distal side cylindrical portion 141 closer to the proximal side than the most distal end 141a flows between the shaft 110 and the cylindrical member 160 as illustrated in FIGS. 8B and 8C. Specifically, in FIG. 8B, the distal portion 141d of the distal side cylindrical portion 141 of the balloon 140 extends to the axial distal end and extends toward a radially inside. The most distal end 141a of the distal side cylindrical portion 141 is positioned radially more inside than an outer surface of the cylindrical member 160. On the other hand, a distal side branch portion 141d1 extends toward a radially inside, and is disposed in a manner of abutting against the outer surface of the inner layer 124 of the shaft 110 (a boundary with another member on a radially outside). The most distal end 124a of the inner layer 124 of the shaft 110 is positioned such that the most distal end 124a enters the cylindrical member 160 on the axial distal side more than the most distal end 141a of the distal side cylindrical portion 141. The most distal end 126a of the outer layer 126 of the shaft 110 is in contact with the distal side branch portion 141d1 of the distal side cylindrical portion 141. On the other hand, in FIG. 8C, the distal portion 141d of the distal side cylindrical portion 141 of the balloon 140 extends in both directions of the axial direction and the radially inside direction as in FIG. 8B, but the distal side branch portion 141d1 extending toward a radially inside has a shape of wrapping the vicinity of the most distal end 124a of the inner layer 124. A distal side branch terminal end 141g of the distal side branch portion 141d1 is located on the surface of the guide wire lumen 121. The distal side of the most distal end 126a of the outer layer 126 is in contact with the inner layer 124. The inner layer 124 has the most distal end 124a covering the vicinity of the most distal end 126a of the outer layer 126. The inner layer 124 further extends in directions of the axial proximal side and the radially outside. An inner layer terminal end 124f of the inner layer 124 is interposed between an inner surface of the distal portion 141d of the distal side cylindrical portion 141 and an outer surface of the outer layer 126.

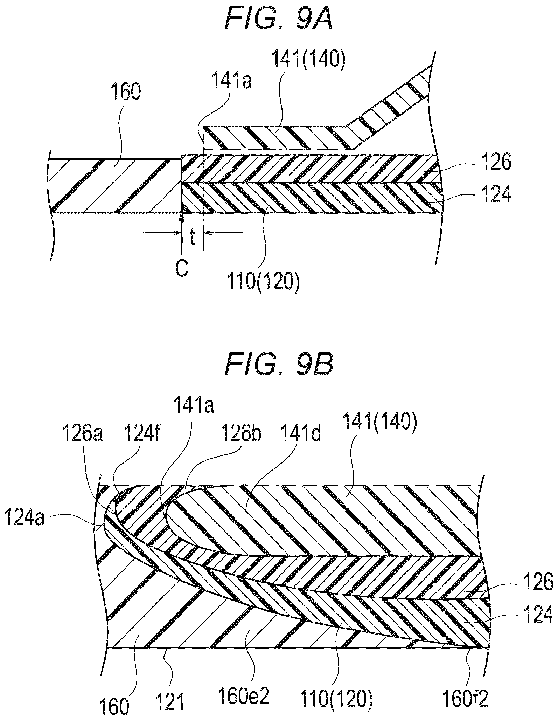

FIG. 9A is a view illustrating a state in which the most distal end 141a of the distal side cylindrical portion 141 of the balloon 140 is positioned closer to the proximal side than the attach position C between the proximal end of the cylindrical member 160 and the distal end of the shaft 110, that is, a state in which the covering length t of the distal side cylindrical portion 141 of the balloon 140 on the cylindrical member 160 is negative (the covering length t < 0) when the cylindrical member 160, the shaft 110, and the balloon 140 are welded at the same time. FIG. 9B is a cross-sectional view illustrating the vicinity of the distal portion of the balloon catheter 100 manufactured by laser-welding the cylindrical member 160, the shaft 110, and the balloon 140 in an arrangement aspect illustrated in FIG. 9A under different manufacturing conditions or using different member materials.

In the balloon catheter 100, as illustrated in FIG. 9A, when the covering length t of the distal side cylindrical portion 141 of the balloon 140 on the cylindrical member 160 is negative, it is possible to manufacture a balloon catheter having a structure in which the shaft 110 flows in a manner of being interposed between the cylindrical member 160 and the distal side cylindrical portion 141 as illustrated in FIG. 9B. Specifically, in FIG. 9B, the outer layer 126 of the first tube shaped body 120 of the shaft 110 covers the most distal end 141a of the distal side cylindrical portion 141 on the axial distal side. The outer layer 126 extends in directions of a radially outside and an axial proximal side via the most distal end 126a, and has an outer layer outer surface 126b that is the outer surface of the balloon catheter 100. The inner layer 124 of the first tube shaped body 120 of the shaft 110 extends between an inner surface of the outer layer 126 and a proximal end surface of the cylindrical member 160, and after the most distal end 124a of the inner layer 124, a thickness in the radial direction of the inner layer 124 gradually decreases, and disappears at the inner layer terminal end 124f. The guide wire lumen side proximal portion 160e2 of the cylindrical member 160 is positioned between an inner surface of the inner layer 124 and the surface of the guide wire lumen 121. A thickness in the radial direction of the guide wire lumen side proximal portion 160e2 decreases toward the axial proximal end, and disappears at the guide wire lumen side proximal end 160f2. The most distal end 141a of the distal side cylindrical portion 141 is positioned closer to the proximal side in the axial direction than the most distal end 124a of the inner layer 124 and the most distal end 126a of the outer layer 126.

As described above, the balloon catheter 100 according to the first embodiment includes the cylindrical member 160, and the balloon 140 that is disposed on the proximal side of the cylindrical member 160 and that includes the distal side cylindrical portion 141, the actuation portion 143 having the space portion 140a inflatable in a cylindrical shape, and the distal side tapered portion 142 connected between the distal side cylindrical portion 141 and the actuation portion 143, and in the axial cross-sectional view, the most distal end 141a of the distal side cylindrical portion 141 is positioned radially more inside than the outer surface 161 of the cylindrical member 160.

Since the most distal end 141a of the distal side cylindrical portion 141 is positioned radially more inside than the outer surface 161 of the cylindrical member 160 in the balloon catheter 100, a joining area of the distal side cylindrical portion 141 to the cylindrical member 160 can be increased. In addition, since the distal portion 141d of the distal side cylindrical portion 141 is formed in a manner of slipping into a thick portion of the cylindrical member 160 after welding, even when the distal portion 141d is bent to follow a shape of a blood vessel, connection with the cylindrical member 160 is firmly maintained. Accordingly, in the balloon catheter 100, since a joining strength between the cylindrical member 160 and the balloon 140 can be improved, separation of the distal portion 141d of the balloon 140 can be prevented.

Next, a balloon catheter 100A according to a second embodiment will be described.

In the following description, differences from the balloon catheter 100 according to the first embodiment will be mainly described in a configuration of the second embodiment. The balloon catheter 100A according to the second embodiment can be made similar to the balloon catheter 100 according to the first embodiment in configuration, manufacturing method, and the like other than the following differences.

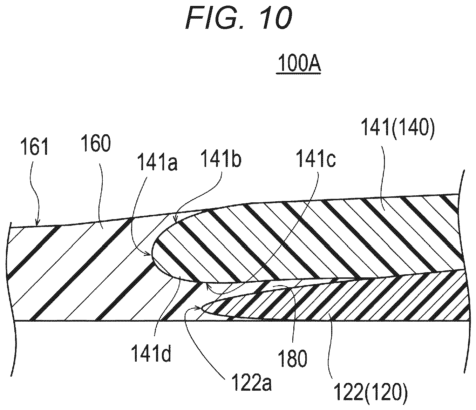

As illustrated in FIG. 10, the balloon catheter 100A includes an interposed portion 180 where a part of the proximal side of the cylindrical member 160 is interposed between the distal portion 122 of the first tube shaped body 120 and the distal portion 141d of the balloon 140.

As illustrated in FIG. 10, the interposed portion 180 is a portion where a part of the proximal portion 160e of the cylindrical member 160 is interposed between the distal portion 122 of the first tube shaped body 120 and the distal portion 141d of the distal side cylindrical portion 141 in an axial cross-sectional view. The interposed portion 180 is formed by a part of the proximal portion 160e of the cylindrical member 160 flowing into a gap formed between the distal portion 122 of the first tube shaped body 120 and the distal portion 141d of the distal side cylindrical portion 141. The part of the proximal portion 160e of the cylindrical member 160 is welded in contact with the distal portion 141d of the distal side cylindrical portion 141 and the distal portion 122 of the first tube shaped body 120.

As described above, in the balloon catheter 100A according to the second embodiment, in the axial cross-sectional view, the distal portion 122 of the first tube shaped body 120 and the distal portion 141d of the distal side cylindrical portion 141 form the interposed portion 180 where a part of the cylindrical member 160 is interposed.

In the balloon catheter 100A, since a part of the proximal side of the cylindrical member 160 is interposed at the interposed portion 180 formed by the distal portion 122 of the first tube shaped body 120 and the distal portion 141d of the distal side cylindrical portion 141, it is possible to improve a joining strength of the cylindrical member 160 to the first tube shaped body 120 and the balloon 140.

The balloon catheter 100 according to the first embodiment and the balloon catheter 100A according to the second embodiment described above can have the following aspects.

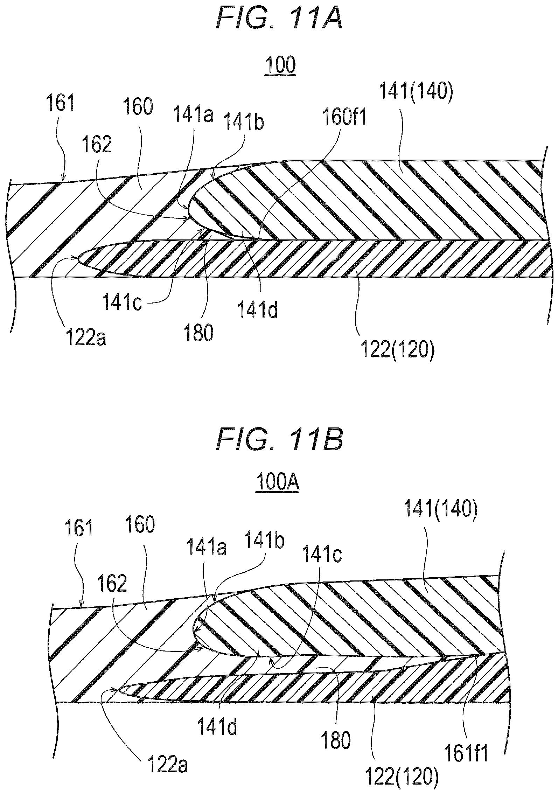

FIG. 11A and FIG. 11B show modifications of the balloon catheter 100 and the balloon catheter 100A.

As illustrated in FIG. 11A, the balloon catheter 100 may be formed such that the most distal end 122a of the distal portion 122 of the first tube shaped body 120 is positioned closer to the distal side than the most distal end 141a of the distal side cylindrical portion 141. In addition, as illustrated in FIG. 11B, the balloon catheter 100A may be formed such that the most distal end 122a of the distal portion 122 of the first tube shaped body 120 is positioned closer to the distal side than the most distal end 141a of the distal side cylindrical portion 141. A difference between aspects illustrated in FIG. 11A and FIG. 11B is an axial length of the interposed portion 180. The axial length of the interposed portion 180 in FIG. 11A is shorter than the axial length of the interposed portion 180 in FIG. 11B. Specifically, in FIG. 11A, in the axial direction, a length from the most distal end 141a of the distal side cylindrical portion 141 to the distal side cylindrical body side proximal end 160f1 is shorter than a length from the most distal end 122a of the distal portion 122 of the first tube shaped body 120 to the most distal end 141a of the distal side cylindrical portion 141. On the other hand, in FIG. 11B, in the axial direction, a length from the most distal end 141a of the distal side cylindrical portion 141 to the distal side cylindrical body side proximal end 161f1 is longer than a length from the most distal end 122a of the distal portion 122 of the first tube shaped body 120 to the most distal end 141a of the distal side cylindrical portion 141.

As described above, when the balloon catheter 100 and the balloon catheter 100A are formed such that the most distal end 122a of the distal portion 122 of the first tube shaped body 120 is positioned closer to the distal side than the most distal end 141a of the distal side cylindrical portion 141, a contact area of the cylindrical member 160 with the first tube shaped body 120 increases, and the distal portion 122 of the first tube shaped body 120 enters the proximal portion 160e of the cylindrical member 160, thereby obtaining an anchor effect. Accordingly, in the balloon catheter 100 and the balloon catheter 100A, a joining strength of the cylindrical member 160 to the first tube shaped body 120 and the balloon 140 can be more effectively improved, and separation of the distal portion 141d of the balloon 140 can be prevented.

In the balloon catheters illustrated in the above-described embodiments, the above-described modification, and in the partial cross-sectional views of FIGS. 5A to 9B, the same configuration and the same effect are obtained even when common configurations are not particularly denoted by reference numerals or description.

## Claims

1. A balloon catheter comprising:
a cylindrical member; and
a balloon that is disposed on a proximal side of the cylindrical member, and that includes a distal side cylindrical portion, an actuation portion having a space portion inflatable in a cylindrical shape, and a distal side tapered portion connected between the distal side cylindrical portion and the actuation portion, wherein
in an axial cross-sectional view, a most distal end of the distal side cylindrical portion is positioned radially more inside than an outer surface of the cylindrical member, and at least the most distal end of the distal side cylindrical portion and a part of a radially inside inner surface that is radially more inside than the most distal end are positioned in contact with the cylindrical member.

2. The balloon catheter according to claim 1, wherein
in the axial cross-sectional view, the cylindrical member includes a boundary portion that covers and is in continuous contact with a distal portion including the most distal end of the distal side cylindrical portion, a radially outside outer surface that is radially more outside than the most distal end, and the radially inside inner surface.

3. The balloon catheter according to claim 2, wherein
the boundary portion includes a curved portion that is continuous in a curved shape via the most distal end of the distal side cylindrical portion.

4. The balloon catheter according to claim 1, wherein
in the axial cross-sectional view, the cylindrical member includes a boundary portion having a curved shape that covers and is in continuous contact with the most distal end of the distal side cylindrical portion and an outer surface distal portion that is radially more outside than the most distal end, and
in a radial direction, a distance between the boundary portion and the outer surface decreases following the curved shape of the boundary portion, and disappears at an outer surface proximal end of the cylindrical member.

5. The balloon catheter according to any one of claims 1 to 4, further comprising:
a first tube shaped body disposed on the proximal side of the cylindrical member, wherein
the space portion is formed by an outer surface of the first tube shaped body and an inner surface of the actuation portion.

6. The balloon catheter according to claim 5, wherein
in the axial cross-sectional view, a distal portion of the first tube shaped body and the distal portion of the distal side cylindrical portion form an interposed portion where a part of the cylindrical member is interposed.

7. The balloon catheter according to claim 5, wherein
a most distal end of the first tube shaped body is positioned closer to a distal side than the most distal end of the distal side cylindrical portion.

8. The balloon catheter according to claim 5, wherein
the most distal end of the distal side cylindrical portion is positioned closer to a distal side than a most distal end of the first tube shaped body.

9. A balloon catheter comprising:
a cylindrical member; and
a balloon that is disposed on a proximal side of the cylindrical member, and that includes a distal side cylindrical portion and an actuation portion having an inflatable space portion, wherein
in an axial cross-sectional view, a distal portion of the distal side cylindrical portion is formed in a tongue shape protruding toward an axial distal side, and the cylindrical member closely covers the distal portion having the tongue shape on a radially inside and a radially outside.

10. The balloon catheter according to claim 9, further comprising:
a first tube shaped body disposed on the proximal side of the cylindrical member, wherein
the space portion is formed by an outer surface of the first tube shaped body and an inner surface of the actuation portion.

11. The balloon catheter according to claim 10, wherein
in the axial cross-sectional view, a distal portion of the first tube shaped body and the distal portion of the distal side cylindrical portion form an interposed portion where a part of the cylindrical member is interposed.
